# EUROPEAN PATENT SPECIFICATION

(11) **EP 2 658 459 B1**
(45) Date of publication and mention of the grant of the patent: **22.04.2015**
(21) Application number: 11810803.4
(22) Date of filing: 23.12.2011
(51) Int. Cl.: A61B 17/72

(54) **Intramedullary nail with shape memory elements for long bones**
Marknagel mit Formgedächtniselementen für lange Knochen
Clou intramédullaire à éléments de mémoire de forme pour os allongés

(30) Priority: 31.12.2010 EP 10197453
(43) Date of publication of application: 06.11.2013
(73) Proprietor: Orthofix S.r.l., 37012 Bussolengo (VR) (IT)
(72) Inventor: COATI, Michele, 37029 San Pietro in Cariano (Verona) (IT); BAGNASCO, Mara, 20134 Milano (IT); ROSSI, Luigi, 37019 Peschiera del Garda (Verona) (IT); MARINI, Graziano, 37060 Castel d'Azzano VR (IT); ROSSI, Graziano, 37139 (Verona) (IT)
(74) Representative: Botti, Mario
(86) International application number: PCT/EP2011/006551
(87) International publication number: WO 2012/089330

(56) References cited:
- WO-A1-2005/094706
- DE-U1-202005 020 788
- US-A- 6 120 504

## Description

### Field of application

The present invention refers, in its most general aspect, to an intramedullary nail suitable to be inserted into a fractured elongated bone, for example a femur or a tibia, and comprising a cannulated rod extending between a proximal end and a distal end.

More particularly, the invention relates to a nail comprising:
- a cannulated rod extending between a proximal end and a distal end;
- an outside tubular sleeve for housing said rod, the rod being coaxial with the sleeve and axially guided inside the tubular sleeve;
- shape-memory elements housed in corresponding seats on said rod; each of the elements being able to assume a configuration in which it is retractably housed in its respective seat, so as to allow the insertion of the nail in the bone, and another configuration in which said elements project from slots in said sleeve.

### Prior art

Intramedullary nails are known in the field and used in surgical interventions, where they are inserted into a fractured elongated bone to return consistency to the bone and allow the mechanism of bone callus regeneration to take place correctly

These nails comprise a stem or rod having a cylindrical shape and they are either solid or hollow; in the latter case the rod is cannulated.

In order to fix the intramedullary nail to the bone portions that are to be reconstructed, usually two offset holes are provided on the nail, with axes lying on parallel planes and extending diametrically across the rod in correspondence with the distal end of the nail. Generally two further offset holes are provided in correspondence with the proximal end of the nail with axis lying on parallel planes.

These holes are intended to receive bone screws inserted into the bone after the appropriate holes have been drilled in the bone, in this manner fastening the intramedullary nail inside the fractured bone.

Although still widely used these commonly known nails have known drawbacks as a result of the fact that holes have to be drilled for the insertion of bone screws. These holes have to be in correspondence with the holes of the inserted intramedullary nail, but since the distal nail holes are not visible, X-ray techniques are needed, causing cumulative exposure to X-ray of the operating staff and great inconvenience during the surgical operation.

More recently alternative nail structures have been proposed. European patent No. EP 1 740 113 and International Publication No. WO 2005/094706A1 owned by the same Applicant, for instance, disclose an intramedullary nail that can be inserted into a fractured elongate bone, comprising a straight stem extending between a proximal end and a distal end and further comprising a plurality of elements made of at least a shape-memory material. The free end of these shape-memory elements are positioned on the outside of the stem for fixation of the nail to the bone.

Whilst advantageous from many points of views, such nails with shape-memory elements also have some drawbacks that are still to be overcome.

For instance, due to the absence of bone screws, there is no complete stability of the nail inside the medullary canal and against the high axial stresses caused by a patient's body weight. When this kind of stress is applied, the nail becomes unstable and may even compromise the process of osteosynthesis or cause anomalies in the healing process.

The technical problem of the present invention is that of providing an intramedullary nail with structural and functional features that overcome all the drawbacks of the prior art solutions through a simpler nail construction for housing the shape-memory elements.

Another object of the nail of the present invention is that of providing a nail that improves the stabilization phase before a final stable configuration is reached during the nail installation.

A further object of the present invention is that of providing an intramedullary nail that will be easier to remove when the bone fracture is fully consolidated.

### Summary of the invention

The idea at the basis of the present invention is that of providing an intramedullary nail to be inserted into a fractured elongated bone according to claim 1.

The dependent claims outline preferred and particularly advantageous embodiments of intramedullary nail according to the invention.

Further features and advantages of the intramedullary nail according to the invention shall become clearer from the following description of an example embodiment thereof, given by way of illustrative and nonlimiting purposes and with reference to the attached drawings.

### Brief description of the drawings

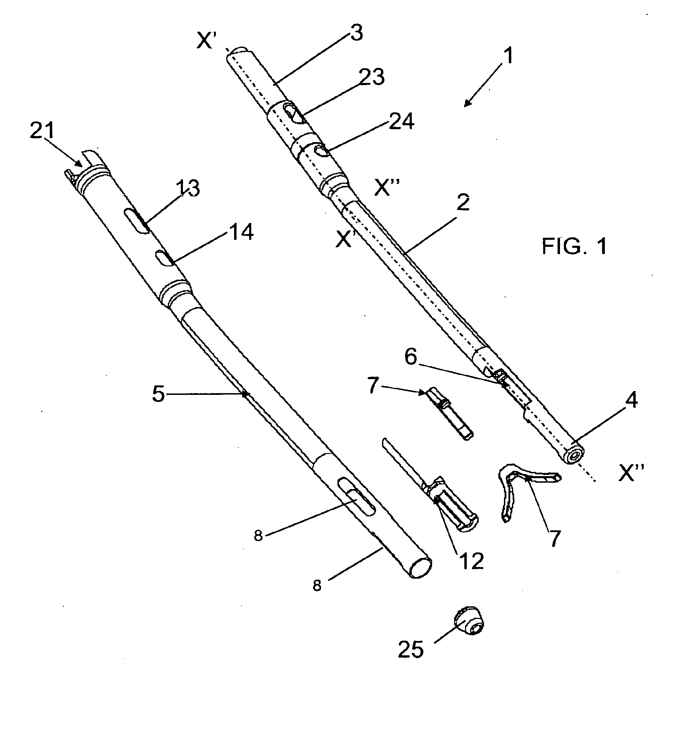

Figure 1 illustrates a perspective view of a nail according to the invention split into its components;
Figure 2 illustrates an elevation view of a tubular sleeve of the nail of figure 1;
Figure 3 illustrates an elevation view of a cannulated rod of the nail of figure 1;

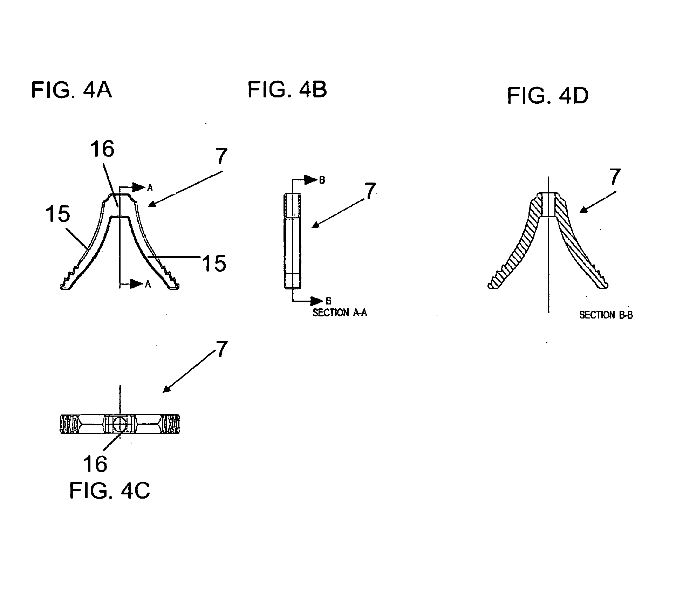

Figures 4A-4D illustrate front, side, top and cross-section views of a shape-memory element incorporated in the nail of the present invention;

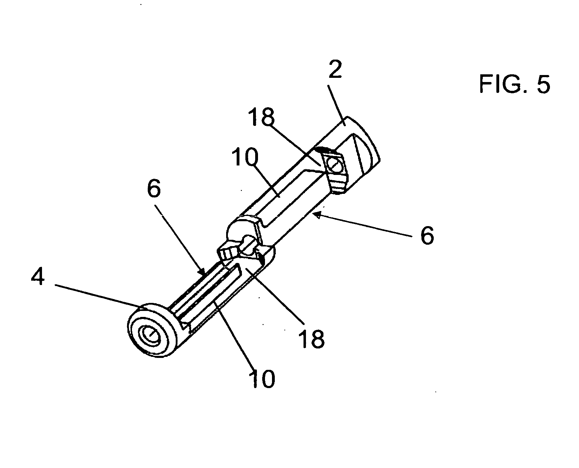

Figure 5 illustrates a perspective view of a detail of a distal end portion of the cannulated rod of figure 3 with seats for the shape-memory elements of figures 4A-4D;

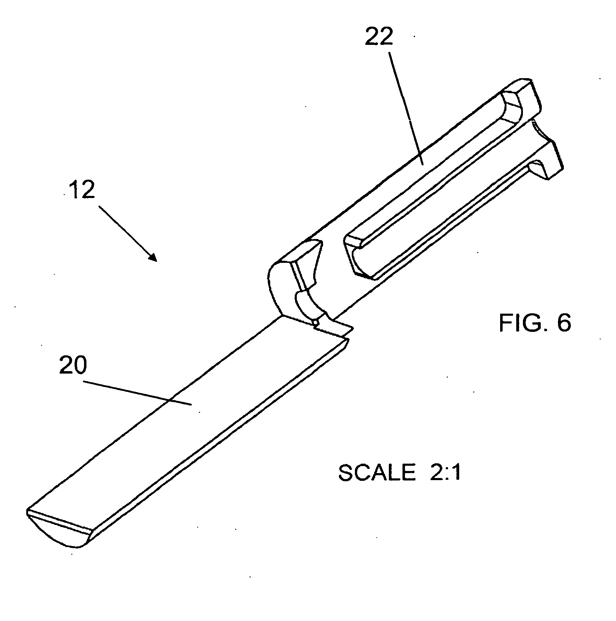

Figure 6 is a perspective view of a distal cover of the cannulated rod shown in figure 3;

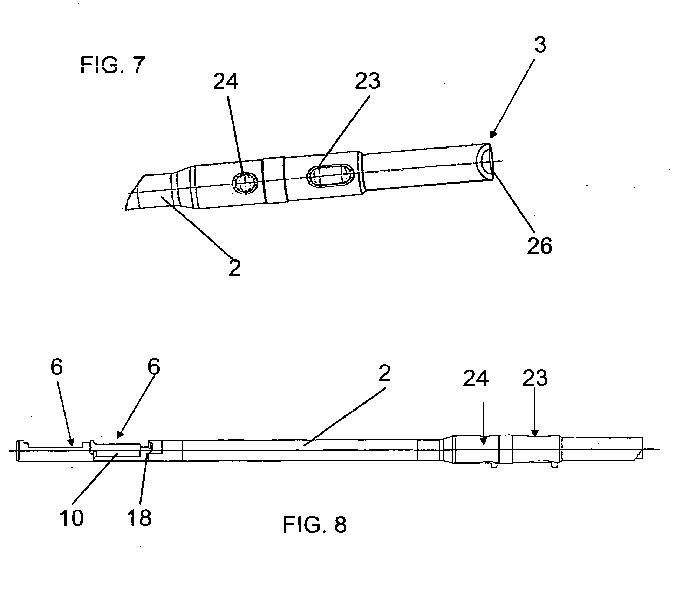

Figure 7 is a side view of a detail of a proximal end portion of the cannulated rod of figure 3;
Figure 8 is a side elongated view of the cannulated rod of figure 3 with the distal cover removed;

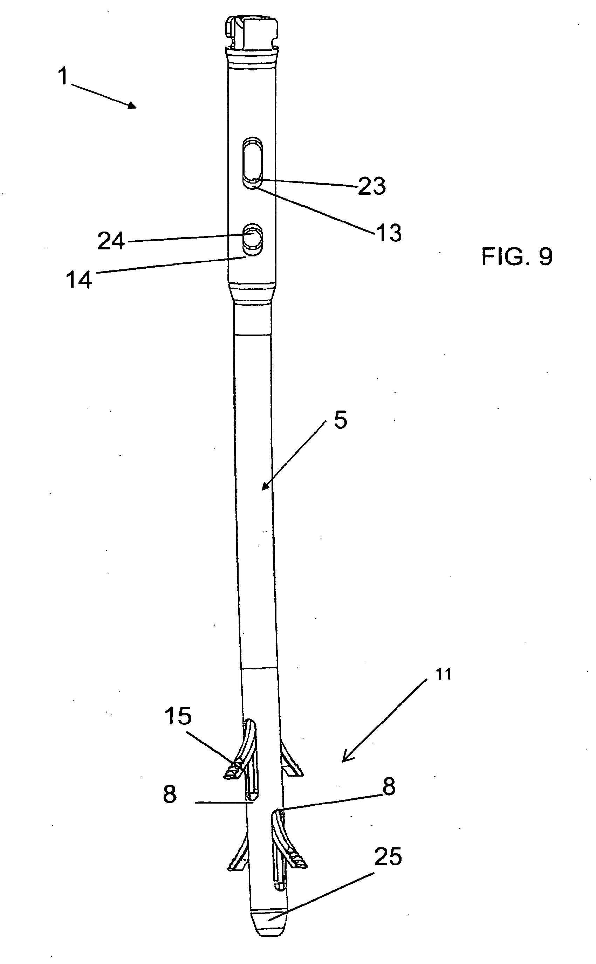

Figure 9 is an elevated front view of the nail of the present invention in its assembled configuration.

### Detailed description

With reference to the aforementioned figures, 1 generally indicates an intramedullary nail in accordance with the present invention, intended to be inserted into a fractured elongated bone, for instance a femur.

In case of a femoral nail, the nail 1 is provided with an anatomic shape having a proximal 5° curvature to follow the shape of the femoral bone.

The nail 1 comprises an inner rod 2 extending between a proximal end 3 and a distal end 4.

The nail 1 further comprises an outside tubular sleeve 5 housing the rod 2 that is inserted coaxially into the tubular sleeve 5.

Consequently, the nail 1 and its main components, the rod 2 and the sleeve 5 are curved or bent so that the axis x'-x' of the proximal end portion forms a small angle of 5° with the axis x"-x" of the remaining body and distal end portions of the nail 1.

Preferably, the nail 1 and its main components, the rod 2 and the sleeve 5 are cylindrical.

Both the sleeve 5 and the rod 2 comprise a pair of transversal holes in the proximal portion of both components; these holes are intended to receive bone screws and have their respective axes for the insertion of the bone screw lying on a common plane.

More specifically, the sleeve 5 comprises a couple of transversal holes or slots 13 and 14 that are provided transversally with respect to the axis of the nail to allow the passage of bone screws.

The more proximal slot 13 is axially longer that the other slot 14.

Since the sleeve 5 is tubular, the slots 13, 14 are provided in opposite and coaxial portions of the perimeter wall of the proximal portion of the sleeve. Both slots 13, 14 have their corresponding through holes parallel to each other.

Further slots 8 are provided in the distal portion of the sleeve 5. These distal slots are angularly offset by 90° with respect to each other and respectively angularly offset by +45° and -45° with respect to the proximal slots 13, 14.

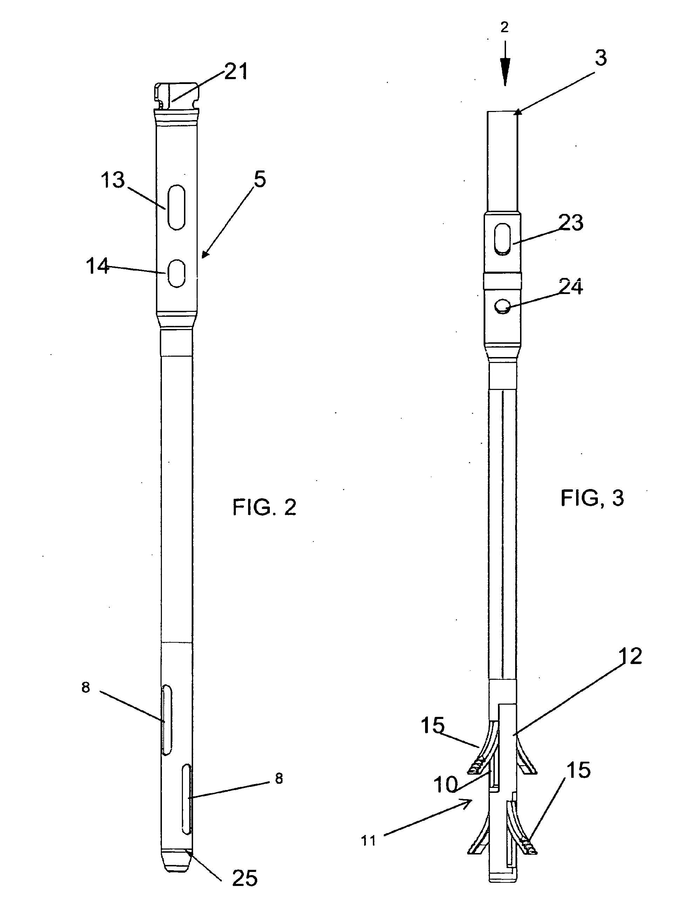

The rod 2 shown in figure 3 is cannulated, that is to say it has an internal cavity that extends longitudinally. The rod may be cannulated along its entire length, as a feature that is used to house a guide wire, for example a Kirschner wire, for use during the insertion into the bone medullary canal.

In accordance with the present invention the inner rod 2 is housed slidingly inside the tubular sleeve 5. More specifically, the rod 2 is axially movable inside the sleeve 5 and driven by an external device attached to the proximal end of the nail 1.

The rod 2 comprises a couple of proximal transversal passages 23, 24 in the proximal portion.

The more proximal passage 23 is a slot that substantially corresponds in shape and extension with the slot 13 of the sleeve 5. The other passage 24 is a through-hole that receives a stabilization screw and this passage has its axis corresponding to the axis of the slot 14 of the sleeve 5 when the rod 2 is in the right position inside the sleeve 5.

What is important is that the relative axially guided movement of the rod 2 inside the sleeve 5 can place the slots 13, 14 in the sleeve 5 to coincide with the corresponding passages 23 and 24 in the rod 2.

In a head portion of the rod 2 a threading 26 is provided for a threaded connection with a suitable driving tool, not shown, for gripping the rod 2 of the nail 1.

In order to allow easy insertion of the nail 1 in the medullary canal an external instrument is foreseen that may be attached to the proximal end of the tubular sleeve 5. To make this connection possible, the proximal end of the tubular sleeve 5 comprises an attachment system 21, for instance of the bayonet type; however, any other fastening system known in the field can be used.

The rod 2 comprises some distal seats 6 for receiving active elements 7 made of at least a shape-memory material.

The active elements 7 are preferably similar to each other.

Each element 7 has substantially a fork-like shape with two little wings 15 connected by a central portion or core 16.

The seats 6 are in the form of a passing opening 18 for housing the core 16 of the active elements 7 and two lateral recesses 10 for housing the wings 15 of the active elements when in the retracted position.

Because of their shape-memory feature the elements 7 can assume different configurations, from a first configuration in which they are fully hidden inside their seat 6 to an extended configuration in which they protrude from the seats of the rod projecting from the corresponding slots 8 provided in the sleeve 5.

In other words, the active elements 7 are structured and suitable to take a first shape or configuration, in which each of the elements 7 is retractably housed in its respective seat 6, with the wings 15 housed by the recesses 10, so as to allow the insertion of the nail into the bone. Another shape or configuration is taken in which said wings 15 of the active elements 7 project from the respective recesses 10 of the seats 6 to abut against and grip the internal wall of the medullary canal of the fractured bone.

With the term "shape-memory material" we mean a material, known in the art, that has a given starting shape and that takes, under predetermined external conditions or when undergoing a predetermined activation condition, also called "material instruction", a given new shape but that returns to the initial shape when the material instructions are deactivated.

For the meaning of the present invention, the starting shape may correspond to the configuration in which the shape-memory elements 7 are arranged projecting from the seats 6 of the rod 2. However, according to the material used, the starting shape might even correspond to the configuration in which the elements 7 are retracted and housed into the seats.

It must be noted that, while the shape-memory elements 7 may be realized with Shape-Memory Alloy, it is preferable to use SIM materials, i.e. materials sensible to Stress-Induced Martensite, for instance Nitinol, an alloy of Nickel and Titanium.

Another characteristic of the shape-memory material lies in the fact that the transition from the first to the second shape, or configuration, is reversible, i.e. the shape-memory elements can be change from the second to the first shape, or configuration, allowing the extraction of the nail from the bone.

The nail 1 of the invention is structured with a pair 11 of elements 7 located at the distal end 4 of the nail 1. We will call this pair 11 of shape-memory elements 7 the distal pair.

Advantageously a first shape-memory element 7 and a second shape-memory element 7 forming the distal pair 11 lie on an offset plane with respect to each other and also both the first and the second shape-memory elements 7 of the distal pair 11 lie on a respective offset plane with respect to the common plane on which the transversal passages 13, 14 and the holes 23, 24 lie.

More particularly, one shape-memory element 7 of the distal pair 11 is oriented at +45° and the other shape-memory element 7 of the distal pair 11 is oriented at -45° with respect to the common plane on which the axis of the slots 13, 14 and passages 23, 24 lie.

Therefore, the first and second element 7 of the distal pair 11 are angularly spaced by 90° from one another.

The fact that the distal elements 7 are offset with one another, for example with an offset of 90° sexagesimal, ensures a determined stability on orthogonal planes and it is useful for granting the nail 1 a better grip inside the medullary canal.

Advantageously, the shape-memory elements of the present invention are structurally independent from the rod 2 and are housed in their corresponding seats 6 of the distal portion of rod 2 but firmly kept in these seats by a distal cover 12. The central core 16 of each element 7 is firmly kept in its seat 6 by the cover 12, while the wings 15 of the elements 7 are freely movable through the openings between the cover 12 and the rod 2 and through the slot 8.

The distal cover 12 has a particular configuration that cooperate with the distal end of the rod to be covered.

The cover 12 is fixed to the distal rod portion of the uncovered rod by soldering or other fixing techniques.

The cover 12, once fixed to the rod, might be considered forming a common piece forming the rod 2 so that each seat 6 is formed by an opening 18 for receiving the core 16 of a shape-memory element 7 and by a couple of opposite recesses 10 for receiving each wing 15 of the same shape-memory element 7 when in the retracted configuration.

In more detail, the distal cover 12 is formed by two portions 20 and 22 that are connected at their respective end forming a single piece and each portion lies on a respective plane which is perpendicular with respect to the other.

Both portions 20, 22 have a concave outside surface that continues the cylindrical surface of the rod 2 when the distal cover is mounted and fixed to the rod 2 to keep the distal pair 11 of shape-memory elements 7 in position.

One of the two portions, numbered 20, of the distal cover 12 is internally flat while the other portion 22 has an elongated internal element having a shape conformed to cooperate with a corresponding internal element provided on the distal end 4 of the rod 2.

The coupling between the distal cover 12 and the distal end of the rod 2 guarantees the continuity of the cylindrical external surface of the cannulated rod 2.

To facilitate the insertion of the nail 1 into the medullary canal of the fractured bone, the tip portion of the sleeve 5 is preferably rounded so as to allow the nail to slide smoothly into said medullary canal.

More specifically, a structurally independent rounded tip 25 is provided to be fixed to the distal end of the sleeve 5.

The tip 25 is soldered to the distal end of the sleeve 5 once the rod 2 has already been inserted into the sleeve.

It must be noted that such a tip 25 maintains the opening of the cannulated rod 2, which has an open end.

As can be appreciated from what has been described, the intramedullary nail according to the present invention meets the requirements and overcomes the drawbacks mentioned in the introductory part of the present description with reference to the prior art solutions.

A clear advantage of the nail according to the present invention is the fact that only one transversal stabilizing screw provided in the proximal nail portion is needed to stabilize the nail in the medullary canal.

Another advantage of the combination of proximal screws with distal shape-memory elements is the fact that a nail arranged in this manner has the double advantages of nails made of shape-memory material and classic nails bone screws.

Of course, a person skilled in the art can apply numerous modifications and variants to the intramedullary nail described above, in order to satisfy contingent and specific requirements. The scope of protection of the invention is defined by the following claims.

## Claims

1. Intramedullary nail to be inserted into a fractured elongated bone and comprising:
a cannulated rod (2) extending between a proximal end (3) and a distal end (4);
an outside tubular sleeve (5) for receiving said rod (2), the rod (2) being coaxial with the sleeve (5) and axially guided inside the tubular sleeve (5);
shape-memory elements (7) housed in corresponding seats (6) of said rod (2); each of the shape-memory elements (7) able to assume a configuration of rest in which said shape-memory elements (7) are housed in their respective seats (6) to allow the nail to be inserted into the bone, and a configuration of use in which said shape-memory elements (7) are projecting from slots (8) in said sleeve (5); wherein
- a distal pair (11) of said shape-memory elements (7) is provided at the distal end (4) of the rod (2); and
- a first shape-memory element (7) and a second shape-memory element (7) forming the distal pair (11) lie on an offset plane with respect to each other
**characterized in that**
- a pair of transversal passages (23, 24) is provided in the proximal portion of said rod (2) and a pair of corresponding transversal holes (13, 14) is provided in the proximal portion of said sleeve (5), said holes and passages being intended to receive bone screws and having their respective axes for the insertion of the bone screw lying on a common plane; and
- both the first and the second shape-memory elements (7) of the distal pair (11) lie on a respective offset plane with respect to said common plane on which said transversal passages and holes (13, 14; 23, 24) lie, said first and second shape-memory elements (7) being retained in their corresponding seats (6) by a distal cover (12) fixed to the rod (2).

2. Intramedullary nail according to claim 1, wherein the first and the second shape-memory element (7) of the distal pair (11) are angularly spaced at 90° from one another.

3. Intramedullary nail according to claim 1 or 2, wherein one shape-memory element (7) of the distal pair (11) lies on a plane at +45° with respect to the plane of said transversal passages and holes (13, 14; 23, 24) while the other element of the distal pair (11) lies on a plane at -45° with respect to the plane of said transversal passages and holes (13, 14; 23, 24).

4. Intramedullary nail according to any one of the previous claims, wherein said transversal holes (13, 14) of the proximal portion of the sleeve (5) are slots on the wall of the sleeve (5).

5. Intramedullary nail according to any one of the previous claims, wherein said transversal passages (23, 24) of the proximal portion of the rod (2) are a through-hole (24) and a through-slot (23).

6. Intramedullary nail according to any one of the previous claims, wherein each shape-memory element (7) has substantially a fork-like shape with two wings (15) connected by a central portion (16).

7. Intramedullary nail according to any one of the previous claims, wherein said shape-memory elements (7) are realized by SIM (Stress Induced Martensite) materials.

8. Intramedullary nail according to claim 7, wherein said SIM material is an alloy of Nickel and Titanium, such as Nitinol.

9. Intramedullary nail according to any one of the previous claims, wherein the distal cover (12) is formed by two portions (20, 22) that are connected at their respective ends forming a single piece and each portion lies on a respective plane which is perpendicular with respect to the other.

10. Intramedullary nail according to claim 9, wherein both said portions (20, 22) have an outside concave surface that continues the cylindrical surface of the rod (2) when the distal cover is mounted and fixed to the rod (2) to retain the distal pair (11) of shape-memory elements (7).

11. Intramedullary nail according to any one of the previous claims, wherein a rounded tip (25) is fixed to the distal end of said sleeve (5).

12. Intramedullary nail according to claim 11, wherein said rounded tip (25) has an open end.

## Patentansprüche

1. Marknagel zum Eingebrachtwerden in einen gebrochenen, länglichen Knochen und umfassend:
einen kanülierten Stab (2), der sich zwischen einem proximalen Ende (3) und einem distalen Ende (4) erstreckt;
eine äußere röhrenförmige Hülse (5) zum Aufnehmen des Stabes (2), wobei der Stab (2) koaxial zu der Hülse (5) ist und axial innerhalb der röhrenförmigen Hülse (5) geführt ist;
Formgedächtniselemente (7), die in zugehörigen Aufnahmen (6) des Stabes (2) untergebracht sind; wobei jedes der Formgedächtniselemente (7) geeignet ist, eine Ruhekonfiguration anzunehmen, in der die Formgedächtniselemente (7) in ihren jeweiligen Aufnahmen (6) untergebracht sind, um es dem Nagel zu ermöglichen, in den Knochen eingebracht zu werden, und eine Verwendungs-Konfiguration, bei der die Formgedächtniselemente (7) aus Schlitzen (8) in der Hülse (5) herausragen; wobei
- ein distales Paar (11) der Formgedächtniselemente (7) an dem distalen Ende (4) des Stabes (2) bereitgestellt ist; und
- ein erstes Formgedächtniselement (7) und ein zweites Formgedächtniselement (7), welche das distale Paar (11) bilden, in einer versetzten Ebene zueinander liegen **dadurch gekennzeichnet, dass**
- ein Paar von transversalen Durchgängen (23, 24) in dem proximalen Abschnitt des Stabes (2) bereitgestellt ist und ein Paar von zugehörigen transversalen Löchern (13, 14) in dem proximalen Abschnitt der Hülse (5) bereitgestellt ist, wobei die Löcher und die Durchgänge dafür bestimmt sind, Knochenschrauben aufzunehmen und deren jeweiligen Achsen für die Aufnahme der Knochenschraube in einer gemeinsamen Ebene liegen; und sowohl das erste als auch das zweite Formgedächtniselement (7) des distalen Paares (11) jeweils in einer gegenüber der gemeinsamen Ebene, in welcher die transversalen Durchgänge und Löcher (13, 14; 23, 24) liegen, versetzten Ebene liegen, wobei die ersten und zweiten Formgedächtniselemente (7) in ihren zugehörigen Aufnahmen (6) durch eine distale Abdeckung (12), die an dem Stab (2) befestigt ist, zurückgehalten werden.

2. Marknagel nach Anspruch 1, wobei das erste und das zweite Formgedächtniselement (7) des distalen Paares (11) winklig 90° voneinander beabstandet sind.

3. Marknagel nach Anspruch 1 oder 2,
wobei ein Formgedächtniselement (7) des distalen Paares (11) in einer Ebene mit +45° in Bezug auf die Ebene der transversalen Durchgänge und Löcher (13, 14; 23, 24) liegt, während das andere Element des distalen Paares (11) in einer Ebene mit - 45° in Bezug auf die Ebene der transversalen Durchgänge und Löcher (13, 14; 23, 24) liegt.

4. Marknagel nach einem der vorangegangenen Ansprüche, wobei die transversalen Löcher (13, 14) des proximalen Abschnitts der Hülse (5) Schlitze auf der Wand der Hülse (5) sind.

5. Marknagel nach einem der vorangegangenen Ansprüche, wobei die transversalen Durchgänge (23, 24) des proximalen Abschnitts des Stabes (2) ein Durchgangsloch (24) und ein Durchgangsschlitz (23) sind.

6. Marknagel nach einem der vorangegangenen Ansprüche, wobei jedes Formgedächtniselement (7) im Wesentlichen eine gabelähnliche Form mit zwei Flügeln (15), die durch einen zentralen Abschnitt (16) verbunden sind, aufweist.

7. Marknagel nach einem der vorangegangenen Ansprüche, wobei die Formgedächtniselemente (7) durch SIM (spannungsinduzierter Martensit) -Materialien verwirklicht sind.

8. Marknagel nach Anspruch 7, wobei das SIM-Material eine Legierung aus Nickel und Titan, wie z.B. Nitinol, ist.

9. Marknagel nach einem der vorangegangenen Ansprüche, wobei die distale Abdeckung (12) durch zwei Abschnitte (20, 22) gebildet ist, die zum Formen eines einzigen Stückes an ihren jeweiligen Enden verbunden sind und wobei jeder Abschnitt auf einer entsprechenden Ebene, die rechtwinklig zu der anderen ist, liegt.

10. Marknagel nach Anspruch 9, wobei die beiden Abschnitte (20, 22) eine konkave Außenfläche aufweisen, die die zylindrische Oberfläche des Stabes (2) fortsetzt, wenn die distale Abdeckung auf dem Stab (2) montiert und befestigt ist, um das distale Paar (11) der Formgedächtniselemente (7) zurückzuhalten.

11. Marknagel nach einem der vorangegangenen Ansprüche, wobei eine abgerundete Spitze (25) an dem distalen Ende der Hülse (5) befestigt ist.

12. Marknagel nach Anspruch 11, wobei die abgerundete Spitze (25) ein offenes Ende aufweist.

## Revendications

1. Clou intramédullaire à insérer dans un os allongé fracturé et comprenant :
une tige canulée (2) qui s'étend entre une extrémité proximale (3) et une extrémité distale (4) ;
un manchon tubulaire extérieur (5) destiné à recevoir ladite tige (2), la tige (2) étant coaxiale avec le manchon (5) et étant guidée axialement à l'intérieur du manchon tubulaire (5) ;
des éléments à mémoire de forme (7) logés dans des sièges correspondants (6) de ladite tige (2) ; chacun des éléments à mémoire de forme (7) pouvant prendre une configuration de repos dans laquelle lesdits éléments à mémoire de forme (7) sont logés dans leurs sièges respectifs (6) de façon à permettre l'insertion du clou dans l'os, et une configuration d'utilisation dans lequel lesdits éléments à mémoire de forme (7) font saillie à partir de fentes (8) situées dans ledit manchon (5) ; dans lequel :
- une paire distale (11) desdits éléments à mémoire de forme (7) est prévue au niveau de l'extrémité distale (4) de la tige (2) ; et
- un premier élément à mémoire de forme (7) et un second élément à mémoire de forme (7) qui forment la paire distale (11) se situent dans des plans décalés l'un par rapport à l'autre ;
**caractérisé en ce que** :
- une paire de passages transversaux (23, 24) sont prévus dans la partie proximale de ladite tige (2) et une paire de trous transversaux correspondants (13, 14) sont prévus dans la partie proximale dudit manchon (5), lesdits trous et lesdits passages étant prévus de façon à recevoir des vis à os, et leurs axes respectifs, pour l'insertion de la vis à os, se situant dans un plan commun ; et
- les premier et second éléments à mémoire de forme (7) de la paire distale (11) se situent dans un plan décalé respectif par rapport audit plan commun dans lequel se situent lesdits passages et lesdits trous transversaux (13, 14; 23, 24), lesdits premier et second éléments à mémoire de forme (7) étant retenus dans leurs sièges correspondants (6) par un couvercle distal (12) fixé sur la tige (2).

2. Clou intramédullaire selon la revendication 1, dans lequel les premier et second éléments à mémoire de forme (7) de la paire distale (11) sont espacés l'un de l'autre en formant un angle de 90°.

3. Clou intramédullaire selon les revendications 1 ou 2, dans lequel un élément à mémoire de forme (7) de la paire distale (11) se situe dans un plan à + 45° par rapport au plan desdits passages et desdits trous transversaux (13, 14; 23, 24), tandis que l'autre élément de la paire distale (11) se situe dans un plan à - 45° par rapport au plan desdits passages et desdits trous transversaux (13, 14 ; 23, 24).

4. Clou intramédullaire selon l'une quelconque des revendications précédentes, dans lequel lesdits trous transversaux (13, 14) de la partie proximale du manchon (5) sont des fentes situées dans la paroi du manchon (5).

5. Clou intramédullaire selon l'une quelconque des revendications précédentes, dans lequel lesdits passages transversaux (23, 24) de la partie proximale de la tige (2) sont un trou traversant (24) et une fente traversante (23).

6. Clou intramédullaire selon l'une quelconque des revendications précédentes, dans lequel chaque élément à mémoire de forme (7) présente une forme sensiblement similaire à celle d'une fourche avec deux ailes (15) reliées par une partie centrale (16).

7. Clou intramédullaire selon l'une quelconque des revendications précédentes, dans lequel lesdits éléments à mémoire de forme (7) sont réalisés dans des matériaux SIM (martensite induite sous contrainte).

8. Clou intramédullaire selon la revendication 7, dans lequel ledit matériau SIM est un alliage de nickel et de titane, tel que le Nitinol.

9. Clou intramédullaire selon l'une quelconque des revendications précédentes, dans lequel le couvercle distal (12) est constitué par deux parties (20, 22) qui sont reliées au niveau de leurs extrémités respectives en formant une seule pièce, et les parties se situent dans des plans respectifs qui sont perpendiculaires l'un par rapport à l'autre.

10. Clou intramédullaire selon la revendication 9, dans lequel lesdites deux parties (20, 22) présentent une surface concave extérieure qui continue la surface cylindrique de la tige (2) lorsque le couvercle distal est monté et fixé sur la tige (2) de façon à retenir la paire distale (11) des éléments à mémoire de forme (7).

11. Clou intramédullaire selon l'une quelconque des revendications précédentes, dans lequel un bout arrondi (25) est fixé sur l'extrémité distale dudit manchon (5).

12. Clou intramédullaire selon la revendication 11, dans lequel ledit bout arrondi (25) présente une extrémité ouverte.
